# EUROPÄISCHE PATENTANMELDUNG

(11) **EP 1 731 617 A1**
(43) Veröffentlichungstag der Anmeldung: **13.12.2006**
(21) Anmeldenummer: 05012131.8
(22) Anmeldetag: 06.06.2005
(51) Int. Cl.: C12P 25/00

(54) **Rückführung lysierter Biomasse als Nährmedium bei der fermentativen Herstellung von Riboflavin**

(71) Anmelder: DSM IP Assets B.V., 6411 TE Heerlen (NL)
(72) Erfinder: Die Erfindernennung liegt noch nicht vor
(74) Vertreter: Schwander, Kuno

(57) **Zusammenfassung**

Die vorliegende Erfindung betrifft ein Verfahren zur Recyclierung von Biomasse bei Fermentationsprozessen, wobei die bei der Herstellung von Fermentationsprodukten anfallende Biomasse mittels eines speziellen Verfahrens aufbereitet und dem System rückgeführt wird. Die aufbereitete Biomasse kann bei der fermentativen Herstellung von Vitaminen, insbesondere Vitamin B2, als Mediumbestandteil bei der Fermentation wieder verwendet werden.

## Beschreibung

Die vorliegende Erfindung betrifft ein Verfahren zur Recyclierung von Biomasse bei Fermentationsprozessen, wobei die bei der Herstellung von Fennentationsprodukten anfallende Biomasse mittels eines speziellen Verfahrens aufbereitet und dem System rückgeführt wird. Die aufbereitete Biomasse kann bei der fermentativen Herstellung von Vitaminen, insbesondere Vitamin B2, als Mediumbestandteil bei der Fermentation wieder verwendet werden.

Viele kommerziell wertvolle Produkte, einschliesslich der Vitamine, werden mittlerweile fermentativ hergestellt. Ein Beispiel dafür ist Vitamin B2 (Riboflavin), welches als essentielles Vitamin für alle Bakterien, Tiere und Pflanzen gilt. Im Gegensatz zu Pflanzen und Bakterien, welche Riboflavin selbst synthetisieren können, muss es bei höheren Tieren, wie z.B. Vertebraten, durch die Nahrung zugeführt werden.

Nach Beendigung einer Fermentation wird normalerweise zunächst das Fermentationsprodukt von anderen Bestandteilen, wie z.B. der Biomasse, isoliert und eventuell weiter aufgereinigt, was zu Verlusten der Ausbeute des Fermentationsproduktes führen kann. Die anfallende Biomasse, obwohl sehr energiereich, bleibt meist als Abfallprodukt ungenutzt und muss unter Aufwand von erheblichen Kosten und Ressourcen entsorgt (gelagert, kompostiert, verbrannt) werden.

Ein weiterer Nachteil der bekannten Fermentationsverfahren sind die hohen Kosten der Fermentation selbst, hervorgerufen durch Zuführen von Rohstoffen zum Kulturmedium. So wird beispielsweise Hefeextrakt zum besseren Wachstum der Mikroorganismen verwendet.

Aufgabe der vorliegenden Erfindung war es, ein verbessertes fermentatives Verfahren zu entwickeln, welches einerseits zur Einsparung von Kosten und Ressourcen führt, andererseits aber auch optimale Ausbeuten und Reinheit des Fermentationsproduktes garantiert.

Es konnte nun überraschenderweise gezeigt werden, dass durch die Verwendung von aufbereiteter Biomasse ("Biomassenrecycling") als Mediumbestandteil bei der Fermentation auf den Zusatz von Hefeextrakt verzichtet werden kann. Ausserdem konnte durch die gewählten Bedingungen während des Aufbereitungsprozesses die Reinheit und Ausbeute des Fermentationsproduktes gesteigert werden.

Die vorliegende Erfindung ist im Speziellen gerichtet auf ein Verfahren zur Recyclierung von Biomasse bei fermentativen Prozessen.

Die Begriffe "Recyclierung von Biomasse" und "recyclierte Biomasse" werden hier verwendet im Sinne von aufbereiteter und rückgeführter bzw. wieder verwendeter Biomasse. Die bei einer Fermentation anfallende Biomasse wird unter geeigneten Bedingungen aufbereitet sodass sie dem System, d.h. dem Fermentationsprozess wieder zugeführt (rückgeführt) werden kann. Die Aufbereitung schliesst dabei Isolierung und Aufschluss sowie gegebenenfalls weitere Reinigungs- und Aufkonzentrierungsschritte ein. Die recyclierte Biomasse kann andere Medienbestandteile, die normalerweise dem Kulturmedium zum besseren Wachstum von Mikroorganismen zugefügt werden, ersetzen. Ein Beispiel eines solchen Bestandteils, der durch die recyclierte Biomasse ersetzt werden kann, ist Hefeextrakt.

Weiterhin betrifft die vorliegende Erfindung ein Verfahren zur fermentativen Herstellung von Fermentationsprodukten, wie z.B. Vitaminen, mittels/unter Verwendung von recyclierter Biomasse.

Das vorliegende Verfahren kann für alle Vitamine verwendet werden, die fermentativ hergestellt werden. Bevorzugte Vitamine sind die Vitamine des B-Komplexes oder Biotin. Bevorzugte Vitamine des B-Komplexes sind Riboflavin, Pantothensäure, Thiamin, Folsäure und Pyridoxin, inklusive ihrer Salzformen und Derivate. Besonders bevorzugt ist Riboflavin, inklusive seine Derivate wie z.B. Riboflavin-Phosphate. Produkte im Sinne dieser Erfindung, d.h. Vitamine hergestellt via Fermentation, werden auch als "Fermentationsprodukte" bezeichnet.

Erfindungsgemässe Fermentationsverfahren zur Herstellung von Vitaminen können im Batch-, Fed-Batch-, kontinuierlichen oder semi-kontinuierlichen Verfahren durchgeführt werden. Das Verfahren kann einschliessen, dass man Zellen von Mikroorganismen, insbesondere rekombinante Mikroorganismen, unter geeigneten Wachstumsbedingungen züchtet, so z.B. durch Animpfen eines Mikroorganismus' in einem geeigneten Medium, dass alle notwendigen Substrate zum Wachstum des Mikroorganismus' und zur Herstellung des Fermentationsproduktes enthält. Das angeimpfte Medium ist bestimmten physico-chemischen Parametern unterworfen, wie z.B. Temperatur, pH und Belüftung, die ein optimales Wachstum der Biomasse und Akkumulation des Fermentationsproduktes erlauben. Je nach verwendetem Mikroorganismus und zu produzierendem Produkt können diese Parameter stark variieren. So kann z.B. die Fermentation unter aeroben oder anaeroben Bedingungen ablaufen. Verfahren zum empirischen Bestimmen dieser Parameter sind dem Fachmann bekannt.

Nach Beendigung der Fermentation kann der resultierende Zellextrakt, enthaltend u.a. Biomasse und das gewünschte (ungereinigte) Fermentationsprodukt, aus der Fermentationsbrühe isoliert und weiter aufgeschlossen werden, z.B. in einem so genannten "Downstream-Processing" weiterverarbeitet werden. Methoden zur Isolierung und Aufreinigung sind dem Fachmann bekannt. So kann das Fermentationsprodukt vom Medium und anderen Bestandteilen, wie z.B. dem Mikroorganismus, isoliert werden. Als "isoliert" versteht man im Sinne dieser Anmeldung, dass z.B. das Produkt gereinigt, oder zumindest teilweise gereinigt ist beispielsweise mittels Filtration, Zentrifitgation und/oder Extraktion. Die anschliessende Reinigung des Produktes kann z.B. erfolgen via Rekristallisierung von wässrigen zu organischen Lösungsmitteln oder durch Anwendung von weiteren dem Fachmann bekannten Methoden, so z.B. Ionenaustausch, Grössenausschluss ("Size-Exclusion") oder Chromatographie via hydrophober Interaktion.

Im Falle von fermentativ hergestelltem Riboflavin, welches in Form von Kristallen ins Kulturmedium ausgeschieden wird, kann die Isolierung aus der Fermentationsbrühe mittels Zentrifugation erfolgen. Die Riboflavinkristalle werden dann in bekannter Weise aufgereinigt, siehe hierzu auch EP 730034 A1 oder Bretzel et al., Journal of Industrial Microbiology & Biotechnology (1999) 22, 19-26.

Die Begriffe "Aufreinigung", "Aufschluss" und "Aufarbeitung" im Zusammenhang mit Zellextrakten einschliesslich Fermentationsprodukten und Biomasse werden in dieser Anmeldung spnonym verwendet.

Bekannte Methoden zur Aufbereitung von Zellextrakten resultierend aus einer Fermentation sind z.B. physikalische, chemische oder biologische Aufschlussmethoden. Ein chemischer Aufschluss beinhaltet z.B. Behandlung mit starken Säuren oder Basen, Lösungsmittel oder Detergenzien. Bei einem physikalischen Aufschluss unterscheidet man zwischen mechanischen, z.B. Ultraschall oder Druck (Hochdruckhomogenisation), und nicht-mechanischen Methoden, wie z.B. osmotischer Schock, Plasmolyse, Einfrieren/Auftauen oder Thermolyse. Biologische Methoden umfassen die Behandlung mit Enzymen, Antibiotika, Phagen oder die Autolyse.

Die erfindungsgemässe Aufarbeitung des Zellextraktes nach erfolgter Fermentation kann mittels physikalischem, chemischem oder biologischem Aufschluss durchgeführt werden. Bevorzugt ist die Autolyse. Der Begriff "Zellextrakt" im Sinne dieser Erfindung bezieht sich auf einen aus einer Fermentation resultierenden Extrakt von Zellen, der ungereinigt vorliegt und u.a. die Biomasse und das Fermentationsprodukt enthalten kann.

Die Aufbereitung des Zellextraktes, z.B. mittels Autolyse, kann direkt an die Fermentation erfolgen, d.h. der Aufschluss erfolgt direkt aus der Fermentationsbrühe. In diesem Falle enthält der aufzubereitende Zellextrakt u.a. Biomasse und das Fermentationsprodukt. Es ist auch möglich, die Biomasse nach Beendigung der Fermentation zunächst mittels dem Fachmann bekannter Methoden aus der Fermentationsbrühe zu isolieren und/oder aufzukonzentrieren, bevor die Aufbereitung der Biomasse, z.B. mittels Autolyse, erfolgt.

In einer bevorzugten Ausführungsform der Erfindung erfolgt die Aufbereitung des Zellextraktes direkt im Anschluss an die Fermentation, d.h. ohne vorherige Abtrennung des Fermentationsproduktes, wie z.B. Riboflavin. Dabei wird die Aufbereitung, bevorzugt Autolyse, direkt im Anschluss an die Fermentation induziert. Die Induktion kann beispielsweise erfolgen durch Änderung der Temperatur.

In einer Ausführungsform der Erfindung wird die Aufarbeitung des Zellextraktes mittels Autolyse durchgeführt. Die Dauer der Autolyse kann in Abhängigkeit des Mikroorganismus und der Bedingungen zwischen ca. 1 h und mehreren Tagen sein, insbesondere ca.8 h, 16 h, oder 24 h. Durch Messung der Zelltrockenmasse (ZTM) kann der Lysegrad ermittelt werden, wobei die Autolyse beendet werden kann, wenn die Werte für die ZTM konstant bleiben. Je niedriger die gemessene ZTM, desto höher der Lysegrad. Methoden zur Messung der ZTM sind dem Fachmann bekannt. Bevorzugt wird die Autolyse für ca. 24 h durchgeführt.

Ein geeigneter pH-Wert bei der Autolyse kann bei ca. 6,0 bis ca. 9,0 liegen, bevorzugt bei ca. 6,0 bis ca. 8,5, insbesondere bei ca, 6,5 bis ca. 8,0. Besonders bevorzugt ist ein pH-Wert von ca. 6,5 bis ca. 7,5. Optimale Resultate werden bei pH-Werten von. 6,5 und 7,5 erreicht.

Eine geeignete Temperatur bei der Autolyse kann im Bereich von ca. 30°C bis ca. 60°C variieren, bevorzugt von ca. 30°C bis ca. 50°C, besonders bevorzugt von ca. 35°C bis ca. 45°C. Optimale Resultate werden bei Temperaturen von ca. 40°C bis ca. 45°C erreicht

In einer Ausführungsform der Erfindung wird die Aufarbeitung des Zellextraktes mittels Autolyse für ca. 24 h bei einem pH-Wert von ca. 6,5 bis ca. 8,0 und bei einer Temperatur von ca. 35°C bis 45°C, durchgeführt.

In einer weiteren Ausführungsform der Erfindung wird die Aufarbeitung mittels Autolyse für ca. 24 h bei einem pH-Wert von ca. 6,5 bis ca. 7,5 und einer Temperatur von ca. 40°C und ca. 45°C durchgeführt.

Zur Optimierung der Aufarbeitung des Zellextraktes kann die Autolyse in Gegenwart von zusätzlichen Enzymen, wie z.B. Hydrolasen oder Proteasen (Endo- und Exoproteasen), durchgeführt werden. Die Menge an Enzym kann beispielsweise 1.5% der ZTM betragen, die Zugabe kann beispielsweise nach ca. 1 bis 5 h ab Beginn der Autolyse erfolgen. Bei ansonsten gleich bleibenden Bedingungen kann die ZTM durch Zusatz dieser Enzyme um bis zu 50% gesenkt werden. Weiterhin führt dies zu erhöhten Lysegraden um bis zu 50%, zu einer Verkürzung der Lysezeiten und zu einer Erhöhung der Produktqualität, d.h. eine höhere Reinheit des Fermentationsproduktes (falls die Autolyse direkt an die Fermentation durchgeführt wird wie oben beschrieben). Nicht-Limitierende Beispiele solcher Proteasen sind Alcalase (Calbiochem), Amano N, Umimazyme, Amano P, Pepdidase R (alle Amano Enzyme Europe Ltd), oder Promod (Biocatalysts Ltd).

In einer Ausführungsform der Erfindung wird die Aufarbeitung mittels Autolyse für ca. 24 h bei einem pH-Wert von ca. 6,5 bis ca. 7,5 und einer Temperatur von ca. 40°C und ca. 45°C unter Verwendung von zugegebenen Proteasen durchgeführt. Bevorzugt ist ein Mix aus Endo- und Exoproteasen.

Die recyclierte Biomasse (aufgearbeiteter Biomassenextrakt) kann bei der fermentativen Herstellung von Vitaminen verwendet werden.

Die vorliegende Erfindung betrifft somit ein Verfahren zur fermentativen Herstellung eines Vitamins, insbesondere Riboflavin, enthaltend (a) die Fermentation eines Mikroorganismus unter Bedingungen und in geeignetem Medium, die die Herstellung des Fermentationsproduktes erlauben, und (b) Aufbereitung der Biomasse, insbesondere durch Autolyse.

Die Biomasse kann aus dem wie oben beschrieben hergestellten Zellextrakt von der Restbiomasse und dem Fermentationsprodukt isoliert/abgetrennt werden und anschliessend weiter aufgereinigt und/oder aufkonzentriert werden. Die Aufreinigung des Lysats kann z.B. durch Crossflowfiltration erfolgen, die Aufkonzentrierung z.B. durch einen Fallstromverampfer. Der so hergestellte aufgearbeitete Biomassenextrakt kann nun in fermentativen Prozessen als Medienbestandteil eingesetzt/wieder verwendet werden.

Im Sinne dieser Erfindung wird ein mittels Autolyse aufbereiteter (Biomassen)Extrakt auch als "Lysat" bezeichnet. Der aufgearbeitete Biomassenextrakt oder das Lysat kann als Ersatz für Hefeextrakt, welches normalerweise bei Fermentationen dem Kulturmedium zum besserten Wachstum beigefügt wird, im Fermentationsprozess eingesetzt werden. Dies bedeutet eine Recyclierung der Biomasse nach entsprechender Aufbereitung wie oben beschrieben. Ausgehend vom Kohlenstoffgehalt von Hefeextrakt, der dem Fachmann bekannt ist, wird der Kohlenstoffgehalt des aufbereiteten Biomassenextraktes, z.B. mittels CHN-Elementaranalysator, bestimmt. Der Hefeextrakt wird somit 1:1 (basierend auf dem Kohlenstoffgehalt) durch den aufbereiteten Biomassenextrakt im Kulturmedium (Fermentationsmedium) ersetzt.

Die vorliegende Erfindung betrifft somit ein Verfahren zur fementativen Herstellung von Vitaminen, insbesondere Riboflavin, enthaltend die folgenden Schritte:
(a) Fermentation eines Mikroorganismus zur Herstellung eines Vitamins, unter Bedingungen und in geeignetem Medium, die die Herstellung des Vitamins erlauben,
(b) Aufbereitung des Zellextrakts, insbesondere durch Autolyse, wobei sich die Aufbereitung direkt an die Fermentation anschliesst,
(c) Isolierung der aufbereiteten Biomasse aus der Fermentationsbrühe enthaltend Vitamin und Restbiomasse,
(d) Aufreinigung und Aufkonzentration der aufbereiteten Biomasse, und
(e) Rückführung des (aufbereiteten) Biomassenextraktes in den Fermentationsprozess unter Wiederholung der Schritte (a) bis (d), wobei die Fermentation ohne Zusatz von Hefeextrakt im Kulturmedium durchgeführt wird.

Bevorzugte Ausführungsformen betreffend Schritte (a) bis (e) sind wie oben beschrieben.

Geeignete Mikroorganismen zur Ausführung der vorliegenden Erfindung können alle Mikroorganismen sein, die sich zur fermentativen Herstellung der oben genannten Produkte eignen, so z.B. für Riboflavin. Die Mikroorganismen könnten ausgewählt werden aus der Gruppe bestehend aus *Escherichia, Bacillus, Cyanobacter, Streptomyces* und Corynebakterien. Es können rekombinante und nicht-rekombinante Mikroorganismen verwendet werden.

Ein Beispiel eines geeigneten Mikroorganismus zur Herstellung von Riboflavin ist *Bacillus.* Bevorzugt ist ein nicht-sporulierender Mikroorganismus der Gattung *Bacillus,* insbesondere *B. subtilis,* besonders bevorzugt *B. subtilis* RB50. Am meisten bevorzugt ist *B. subtilis* RB50::(pRF69)ₙ::(pRF93)ₘ (Perkins et al., J. Ind. Microbiol Biotechnol 22:8-18, 1999).

*B. subtilis* RB 50 wurde bei der Agricultural Research Culture Collection (NRRL), Peoria, IL, USA, am 23.05.1989 nach dem Budapester Vertrag hinterlegt unter der Nummer NRRL B-18502. Plasmid pRF69 wurde am 06.06.1990 bei der American Type Culture Collection (ATCC), Rockville, MD, USA, nach dem Budapester Vertrag hinterlegt unter der Nummer ATCC 68338. Plasmid pRF93 ist beschrieben in EP 0 821 063.

In einer Ausführungsform betrifft die vorliegende Erfindung ein Verfahren zur Herstellung von Riboflavin, enthaltend:
(a) Fermentation eines Mikroorganismus der Gattung *B. subtilis,* unter Bedingungen und in geeignetem Medium, die die Herstellung von Riboflavin erlauben,
(b) Autolyse bei einem pH-Wert von ca. 6,5 bis ca. 7,5 und einer Temperatur von ca. 45°C für ca. 24 h,
(c) Isolierung der aufbereiteten Biomasse aus der Fermentationsbrühe enthaltend Riboflavin und Restbiomasse,
(d) Aufreinigung und Aufkonzentration der aufbereiteten Biomasse, und
(e) Rückführung des *B. subtilis-Lysats* in den Fermentationsprozess unter Wiederholung der Schritte (a) bis (d), wobei die Fermentation ohne Zusatz von Hefeextrakt im Kulturmedium durchgeführt wird.

In einem weiteren Aspekt der Erfindung wird ein Verfahren zur Herstellung von Riboflavin beansprucht, wobei durch Aufbereitung der Fermentationsbrühe mittels Autolyse, wie oben beschrieben, die Ausbeute und Reinheit des erhaltenen Riboflavins gesteigert werden kann.

Im speziellen betrifft die Erfindung ein Verfahren zur Herstellung von Riboflavin, enthaltend:
(a) Fermentation eines Mikroorganismus der Gattung *B. subtilis,* unter Bedingungen und in geeignetem Medium, die die Herstellung von Riboflavin erlauben,
(b) Autolyse der aus Schritt (a) resultierenden Fermentationsbrühe enthaltend fennentativ hergestelltes Riboflavin,
(c) Isolierung des Riboflavin und eventuell
(d) Reinigung des Riboflavins.

Methoden zur Isolierung und Reinigung von Riboflavin sind oben beschrieben und dem Fachmann bekannt. Die Autolyse erfolgt unter Bedingungen wie oben beschrieben. Durch dieses Verfahren können Ausbeutesteigerungen von bis zu 2%, 4%, 6%, 10%, 20% oder 30% erreicht werden, wobei die Riboflavinkonzentrationen vor und nach Schritt (b) gemessen werden. Bevorzugt ist dabei ein Verfahren, bei dem die Autolyse direkt auf die Fermentation folgt.

In einer bevorzugten Ausführungsform wird ein Verfahren zur Herstellung von Riboflavin beansprucht, enthaltend:
a) Fermentation eines Mikroorganismus der Gattung *B. subtilis,* unter Bedingungen und in geeignetem Medium, die die Herstellung von Riboflavin erlauben,
(b) Autolyse der aus Schritt (a) resultierenden Fermentationsbrühe bei einem pH-Wert von ca. 6,5 bis ca. 7,5 und einer Temperatur von ca. 45°C für ca. 24 h,
(c) Trennung der aufbereiteten Biomasse und des Riboflavins von den übrigen Bestandteilen,
(d) Reinigung des Riboflavin sowie Aufreinigung und Aufkonzentration der aufbereiteten Biomasse, und
(e) Rückführung des *B. subtilis*-Lysats in den Fermentationsprozess unter Wiederholung der Schritte (a) bis (d), wobei die Fermentation ohne Zusatz von Hefeextrakt im Kulturmedium durchgeführt wird.

Optional kann die Autolyse durch Verwendung verschiedener Enzyme unterstützt werden, wie oben näher beschrieben.

Ein weiterer Aspekt der Erfindung betrifft die Riboflavin-Synthase, die den letzten Schritt der biosynthetischen Herstellung in *B. subtilis* katalysiert, d.h. die Umwandlung von 6,7-dimethyl-8-ribityllumazine (DMRL) zu Riboflavin. Das oben beschriebene Verfahren führt zu einer Steigerung der Riboflavin-Synthase-Aktivität. Die Aktivität kann beispielsweise durch die Umwandlungsrate von DMRL zu Riboflavin gemessen werden.

Das oben dargestellte Verfahren zur Herstellung von Riboflavin führt weiterhin zur Steigerung der Reinheit des Fermentationsproduktes, was sich z.B. durch einen reduzierten DNA-Gehalt in den Riboflavinkristallen messen lässt. Dies ist insbesondere bei der Herstellung von Riboflavin mittels rekombinanter Mikroorganismen wichtig. Methoden zur Messung des DNA-Gehaltes, wie z.B. PCR, sind dem Fachmann bekannt.

### Beispiele

### Beispiel 1: Kultivierungstechniken und Analyseverfahren

Zur Riboflavinherstellung wurde der *Bacillus subtilis* Stamm RB50::(pRF69)ₙ::(pRF93)ₘ für 48 h in einem Glucose-limitierten Medium mittels Fed-Batch Fermentation gezüchtet (Perkins et al., J. Ind. Microbiol Biotechnol 22:8-18, 1999).

Das Wachstum der Biomasse wurde anhand der Biotrockenmasse (BTM) bestimmt. Die Biotrockenmasse wurde bestimmt, indem die Fermentationsbrühe auf RT (Raumtemperatur) abgekühlt wurde. Anschließend wurden 10 ml in ein vorgewogenes Reagenzglas gegeben und bei 11000g abzentrifugiert. Der Überstand wurde verworfen und das Pellet bei 95°C mindestens 24 h bis zur Gewichtskonstanz getrocknet. Die BTM ergab sich aus der bestimmten GTM (Gesamttrockenmasse) minus dem Gehalt an Riboflavin (Bestimmung des Riboflavingehalts mittels HPLC).

### Beispiel 2: Herstellung eines Extraktes mittels Druck (physikalischer Aufschluss)

Nach Beendigung einer Riboflavin-Fermentation (siehe Beispiel 1) wurde die erhaltene Fermentationslösung abzentrifugiert bei 4250g und die Biomasse isoliert. Die erhaltene Biomasse wurde dreimal mit 50 mM Natrium-Kalium-Phosphatpuffer (pH 7,0) gewaschen und der Proteingehalt sowie die BTM untersucht. Die Biomasse wurde in 3 Durchläufen bei 1900 bar im Hochdruckhomogenisator (Microfluidizer M110-EH, Microfluidcs, Newton, MA, USA) aufgeschlossen (2 Düsensystem 1. Düse: 200 µm; 2. Düse: 100 µm).

### Beispiel 3: Herstellung eines Extraktes mittels Autolyse (biologischer Aufschluss)

Zur Feststellung der optimalen Temperatur der Autolyse wurde die aus Beispiel 1 erhaltene Fermentationslösung mit einem Riboflavin-Gehalt >20 g/l unter Rühren und ohne Einleiten von Luft bei einem konstanten pH-Wert von 7,0 und den in Tabelle 1 angegebenen Temperaturen für 24 h inkubiert. Anschliessend wurde der Lysegrad anhand der BTM bestimmt (s. Tab. 1). Eine optimale Autolyse wurde bei einer Temperatur von 40 - 45°C erreicht.

**Tabelle 1: Autolyse in Abhängigkeit der Temperatur**

| | | | | | | | |
|---|---|---|---|---|---|---|---|
| Temperatur [°C] | 30 | 35 | 40 | 45 | 50 | 55 | 60 |
| BTM [%] | 56,9 | 53,2 | 43,5 | 51,2 | 68,8 | 74,4 | 86,5 |

Zur Feststellung des optimalen pH-Werts der Autolyse wurde die aus Beispiel 1 erhaltene Fermentationslösung mit einem Riboflavin-Gehalt >20 g/l unter Rühren und ohne Einleiten von Luft bei einer konstanten Temperatur von 40°C und den in Tabelle 1 angegebenen pH-Werten für 24 h inkubiert. Anschliessend wurde der Lysegrad anhand der BTM bestimmt (s. Tab. 2). Eine optimale Autolyse wurde bei einem pH-Wert von 6,5 - 7,5 erreicht.

**Tabelle 2: Autolyse in Abhängigkeit des pH-Wertes**

| | | | | | | | | | |
|---|---|---|---|---|---|---|---|---|---|
| pH | 5.0 | 5.5 | 6.0 | 6.5 | 7.0 | 7.5 | 8.0 | 8.5 | 9.0 |
| BTM [%] | 100 | 91,9 | 62.7 | 44,5 | 45,9 | 43,5 | 46,1 | 69,9 | 69,1 |

Aus diesen Resultaten ergibt sich für eine optimale Autolyse eine pH-Wert-Einstellung von 6.5 bis 7.5 bei einer Temperatur von 40 - 45 °C. Um das Risiko einer Kontamination zu minimieren ohne antiseptische Mittel zu verwenden wurde die Temperatur im Weiteren auf 45°C eingestellt.

In einer weiteren Versuchsreihe wurde die Autolyse in Gegenwart verschiedener Proteasen getestet und ihr Einfluss auf den Lysegrad untersucht

Folgende Enzyme wurden verwendet: Alcalase (Calbiochem, San Diego, CA, USA), Amano N, Amano P, Umimazyme, Peptidase R (alle von Amano Enzyme Europe Ltd, Chipping Norton, U.K.) sowie Promod 194P (Biocatalysts Ltd, Treforest, U,K).

Die Autolyse wurde wie oben beschrieben bei einem pH von 6,5 bzw. 7,5 (45°C, 24 h) durchgeführt, mit oder ohne die oben beschriebenen Enzyme gemäss Tabelle 3. Anschliessend wurde der Lysegrad anhand der BTM bestimmt. Eine Steigerung der Lysegrade um max. 20% wurde durch Zugabe von Endoproteasen während des Lyse-Prozesses erreicht (s. Tab. 3).

### Beispiel 4: Herstellung von Riboflavin mittels rückgeführtem Biomasseextrakt

Der gewonnene Extrakt aus Beispiel 3 wurde von Schwebstoffen mittels Crossflowfiltration (cutoff 10 kDa) gereinigt und in der Fermentation wie in Beispiel 1 eingesetzt, wobei das Hefeextrakt 1:1 aufgrund der Kohlenstoffbilanz ersetzt wurde. Hierbei wurden folgende Ergebnisse·erzielt (s. Tab. 3)

**Tabelle 3: Riboflavin-Produktion mittels Rückführung der Biomasse**

| | Riboflavinkonzentration [%] | Ausbeute [%] |
|---|---|---|
| Standard (Hefeextrakt) | 100 | 100 |
| Bacillus-Lysat (aus gewaschener Biomasse | 90,7 | 93,6 |
| Bacillus-Lysat (aus ungewaschener Biomasse) | 78,7 | 77,7 |

## Patentansprüche

1. Verfahren zur Recyclierung von Biomasse bei fermentativen Prozessen enthaltend die Aufbereitung eines aus einer Fermentation resultierenden Zellextraktes zur Rückführung der aufgereinigten Biomasse in Fermentationsverfahren.

2. Verfahren zur fermentativen Herstellung von Vitaminen unter Verwendung von nach Anspruch 1 hergestellter recyclierter Biomasse.

3. Verfahren nach Anspruch 2 wobei die Vitamine ausgewählt sind aus der Gruppe bestehend aus Riboflavin, Pantothensäure, Biotin, Thiamin, Pyridoxin und Folsäure, insbesondere Riboflavin.

4. Verfahren nach einem der vorangehenden Ansprüche wobei die Aufbereitung des Zellextraktes durch physikalische, chemische oder mechanische Lyse erfolgt, insbesondere durch Autolyse.

5. Verfahren nach Anspruch 4 wobei die Autolyse bei einem pH-Wert von ca. 6,0 bis ca. 9,0, insbesondere von ca. 6,0 bis ca. 8,5, und einer Temperatur von ca. 30°C bis ca. 60°C, insbesondere von ca. 30°C bis 50°C, für ca. 24 h durchgeführt wird.

6. Verfahren nach Anspruch 5 wobei die Autolyse bei einem pH-Wert von ca. 6,5 bis ca. 7,5 und einer Temperatur von ca. 40°C bis ca. 45°C für ca. 24 h durchgeführt wird.

7. Verfahren nach einem der vorangehenden Ansprüche wobei die Aufarbeitung der Biomasse mittels Autolyse in Gegenwart von zugegebenen Proteasen durchgeführt wird.

8. Verfahren nach einem der vorangehenden Ansprüche wobei die Aufarbeitung der Biomasse direkt auf die Fermentation folgt ohne Zwischenschalten eines Reinigungsschritts.

9. Verfahren nach einem der vorangehenden Ansprüche enthaltend die folgenden Schritte:
(a) Fermentation eines Mikroorganismus zur Herstellung eines Vitamins, insbesondere Riboflavin, unter Bedingungen und in geeignetem Medium, die die Herstellung des Vitamins erlauben,
(b) Aufbereitung der Biomasse, insbesondere durch Autolyse, wobei sich die Aufbereitung direkt an die Fermentation anschliesst,
(c) Isolierung der aufbereiteten Biomasse aus der Fermentationsbrühe enthaltend Vitamin und Restbiomasse,
(d) Aufreinigung und Aufkonzentration der aufbereiteten Biomasse, und
(e) Rückführung des aufbereiteten Biornassenextraktes in den Fermentationsprozess unter Wiederholung der Schritte (a) bis (d), wobei die Fermentation ohne Zusatz von Hefeextrakt im Kulturmedium durchgeführt wird.

10. Verfahren nach Anspruch 9 zur Herstellung von Riboflavin, wobei Schritt die Aufbereitung in Schritt (b) mittels Autolyse bei einem pH-Wert von ca. 6,5 bis ca. 7,5 und einer Temperatur von ca. 45°C für ca. 24 h erfolgt.

11. Verfahren nach einem der vorangehenden Ansprüche wobei die bei der Fermentation eingesetzten Mikroorganismen ausgewählt sind aus der Gruppe bestehend aus *Escherichia, Bacillus, Cyanobacter, Streptomyces und Corynebacterium,* insbesondere *Bacillus.*

12. Verfahren nach Anspruch 11 wobei der bei der Fermentation eingesetzte Mikroorganismus *Bacillus subtilis* ist, insbesondere ein nicht-sporulierender *B. subtilis.*

13. Verfahren nach Anspruch 12 wobei der bei der Fermentation eingesetzte Mikroorganismus *B. subtilis* RB50 ist, insbesondere RB50::(pRF69)ₙ::(pRF93)ₘ.

14. Verfahren nach einem der vorangehenden Ansprüche 2 bis 13 enthaltend:
a) Fermentation eines Mikroorganismus der Gattung *B. subtilis,* unter Bedingungen und in geeignetem Medium, die die Herstellung von Riboflavin erlauben,
(b) Autolyse der aus Schritt (a) resultierenden Fermentationsbrühe bei einem pH-Wert von ca. 6,5 bis ca. 7,5 und einer Temperatur von ca. 45°C für ca. 24 h,
(c) Trennung der aufbereiteten Biomasse und des Riboflavins von den übrigen Bestandteilen,
(d) Reinigung des Riboflavin sowie Aufreinigung und Aufkonzentration der aufbereiteten Biomasse, und
(e) Rückführung des *B. subtilis-Lysats* in den Fermentationsprozess unter Wiederholung der Schritte (a) bis (d), wobei die Fermentation ohne Zusatz von Hefeextrakt im Kulturmedium durchgeführt wird.
